(19) Europäisches Patentamt
European Patent Office
Office européen des brevets

(11) Veröffentlichungsnummer: **0 006 572**
**B1**

(12) # EUROPÄISCHE PATENTSCHRIFT

(45) Veröffentlichungstag der Patentschrift: **26.08.81**

(51) Int. Cl.³: **C 07 D 321/10,** C 11 B 9/00, A 61 K 7/46

(21) Anmeldenummer: **79102031.6**

(22) Anmeldetag: **20.06.79**

(54) **13,15-Dioxabicyclo (10.5.0) heptadecane, deren Herstellung und Verwendung als Riechstoff sowie diese enthaltende Riechstoffkompositionen.**

(30) Priorität: **26.06.78 DE 2827979**

(43) Veröffentlichungstag der Anmeldung: **09.01.80 Patentblatt 80/1**

(45) Bekanntmachung des Hinweises auf die Patenterteilung: **26.08.81 Patentblatt 81/34**

(84) Benannte Vertragsstaaten: **DE FR GB NL**

(56) Entgegenhaltungen:
**CH-A-435 521**
**CH-A-474 567**
**CH-A-530 460**
**DE-A-2 550 610**
**DE-A-2 407 863**

(73) Patentinhaber: **Henkel Kommanditgesellschaft auf Aktien, -Patentabteilung-**
**Postfach 1100 Henkelstrasse 67,**
**D-4000 Düsseldorf 1 (DE)**

(72) Erfinder: **Bruns, Klaus, Dr., Notburgaweg 6,**
**D-4150 Krefeld-Traat (DE)**
Erfinder: **Meins, Peter, Dr., Karpendeller Weg 19,**
**D-4020 Mettmann (DE)**

**EP 0 006 572 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# 13,15-Dioxabicyclo[10.5.0]heptadecane, deren Herstellung und Verwendung als Riechstoff sowie diese enthaltende Riechstoffkompositionen

Es wurde gefunden, dass 13,15-Dioxabicyclo-[10.5.0]heptadecane der allgemeinen Formel

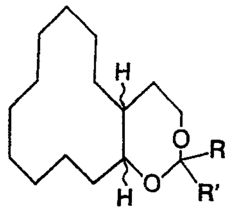

in der R und R' jeweils einzeln oder gemeinsam Wasserstoff oder einen Methylrest darstellen, wertvolle neue Riechstoffe mit warmer Ambra-Note sowie aussergewöhnlicher Haftfestigkeit darstellen.

Als unter die vorgenannte Formel fallende neue Riechstoffe sind 13,15-Dioxabicyclo[10.5.0]heptadecan, 14-Methyl-13,15-Dioxabicyclo[10.5.0]-heptadecan sowie 14-Dimethyl-13,15-Dioxabicyclo[10.5.0]heptadecan zu nennen. Besondere Bedeutung kommt dabei dem unsubstituierten 13,15-Dioxabicyclo[10.5.0]heptadecan zu, da es sich durch eine angenehme, warme Ambranote und eine aussergewöhnliche Haftfestigkeit auszeichnet. Ein weiterer Vorteil der 13,15-Dioxabicyclo{10.5.0}heptadecane ist ihre sehr gute Kombinationsfähigkeit zu neuartigen Kompositionen, denen sie gleichfalls eine besondere Haftfestigkeit verleihen.

Bicyclische Ringsysteme mit cyclischen Acetal- und Ketalstrukturen sind als Riechstoffe an sich bekannt. So werden z. B. in der Schweizer Patentschrift 530 460 Dioxabicyclo{10.3.0}pentadecan und Dioxabicyclo{10.3.0}pentadecadiene als Riechstoffe mit pilziger, erdiger oder holziger Geruchsnote beschrieben. Die Schweizer Patentschrift 435 521 betrifft offene und cyclische Ketale des Cyclododecanons, die in Abhängigkeit von der Struktur der zur Ketalisierung verwendeten Alkohole oder Glykole holzige, zedrige, camphrige, pilzige oder an Patschouli erinnernde Duftnoten aufweisen. Es sind jedoch bisher keine Verbindungen dieses Typs bekanntgeworden, die sich durch warme Ambra-Duftnoten auszeichnen. Auch die in der deutschen Offenlegungsschrift 2 550 610 genannten 13-Oxabicyclo-{10.4.0}hexadec-1(12)en-14-one oder die in der deutschen Offenlegungsschrift 2 407 863 genannten 4,7-Dihydro-1,3-dioxepine oder die in der Schweizer Patentschrift 474 567 genannten Monoepoxide der Trimethylcyclododecatriene zeigen Geruchseigenschaften, die mit den erfindungsgemässen 13,15-Dioxabicyclo{10.5.0}heptadecanen nicht vergleichbar sind.

Die Herstellung der erfindungsgemässen neuen Verbindung 13,15-Dioxabicyclo[10.5.0]heptadecan erfolgt nach an sich bekannten Syntheseverfahren der organischen Chemie. Als Ausgangsmaterial für die Synthese dient Cyclododecanon (I), das mit Bromessigester nach Reformatsky zum entspreechenden Hydroxyester (II) umgesetzt wird, der sich im stark sauren Milieu unter Erhitzen leicht in das Lacton (III) umlagert. Anschliessend wird das Lacton mit Natriumborhydrid in Isopropanol in das Diol (IV) übergeführt, aus dem durch Acetalisierung mit Paraformaldehyd in Äthylenchlorid das 13,15-Dioxabicyclo[10.5.0]-heptadecan (V) als Gemisch zweier Stereoisomere etwa im Verhältnis 1 : 25 erhalten wird. Die Herstellung verläuft gemäss nachstehendem Reaktionsschema:

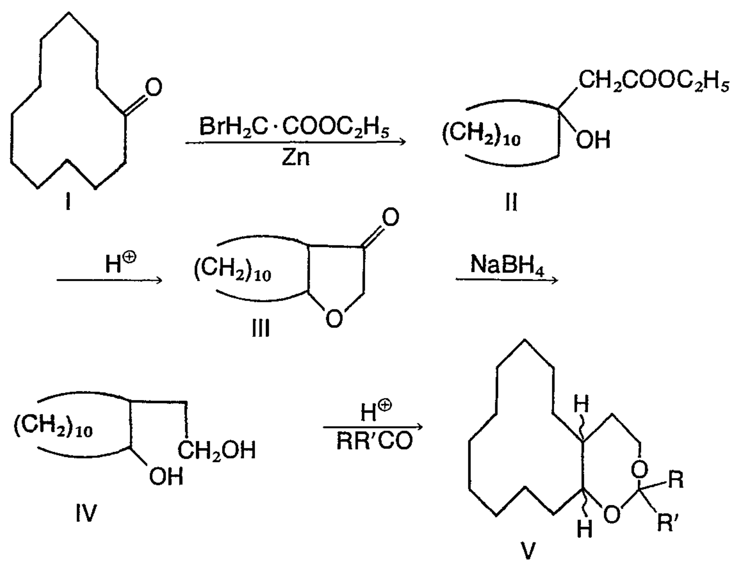


I II III IV V

Die methylsubstituierten 13,15-Dioxabicyclo-[10.5.0]eptadecane sind vom Diol IV aus durch Acetalisierung bzw. Ketalisierung zugänglich.

Die 13,15-Dioxabicyclo[10.5.0]heptadecane können mit anderen Riechstoffen in den verschiedensten Mengenverhältnissen zu neuen Riechstoffkompositionen gemischt werden. Im allgemeinen wird sich ihr Anteil in den Riechstoffkompositionen in den Mengen von 1 bis 50 Gewichtsprozent, bezogen auf die gesamte Komposition, bewegen. Derartige Kompositionen können direkt als Parfüm oder auch zur Parfümierung von Kosmetika, wie Cremes, Lotionen, Duftwässern, Aerosolen, Toiletteseifen usw. dienen. Sie können aber auch zur Geruchsverbesserung technischer Produkte wie Wasch- und Reinigungsmitteln, Weichspülern, Textilbehandlungsmitteln eingesetzt werden. Zur Parfümierung der verschiedenen Produkte werden diesen die Kompositionen im allgemeinen in Konzentrationen von 0,05 bis 2 Gewichtsprozent zugesetzt.

Die nachfolgenden Beispiele sollen den Gegenstand der Erfindung näher erläutern, ohne ihn jedoch hierauf zu beschränken.

Beispiele

Herstellung des 13,15-Dioxabicyclo[10.5.0]heptadecan

a) Herstellung des Hydroxyesters

In eine Suspension von 33 g Zinkpulver in Toluol/Benzol (140/160 ml), das mit einigen Kristallen Jod aktiviert wurde, wurde unter Rühren und Rückfluss während 4 Sunden eine Lösung von 182 g (1 Mol) Cyclododecanon und 167 g (1 Mol) Bromessigsäureäthylester in Toluol/Benzol (140/160 ml) zugetropft und danach weitere 2 Stunden am Rückfluss erhitzt. Das verwendete Zinkpulver wurde in folgender Weise vorbehandelt: Zinkpulver wurde ca. 20 Minuten in 10%iger Salzsäure gerührt, abgesaugt, mit Wasser neutral gewaschen und mit Aceton trokken gewaschen. Anschliessend wurde bei 50 °C im Vakuum getrocknet.

Nach dem Abkühlen des Reaktionsgemisches wurde nicht umgesetztes Zinkpulver unter Rühren und Kühlung mit ca. 300 ml eiskalter Schwefelsäure (10%ig) gelöst, die organische Phase abgetrennt, mit 2n-Sodalösung, 2n-Schwefelsäure und schliesslich mit Wasser neutral gewaschen. Nach dem Trocknen über Natriumsulfat wurde das Lösungsmittel entfernt und der erhaltene Hydroxyester im Ölpumpenvakuum vom nicht umgesetzten Cyclododecanon befreit.

b) Herstellung des Lactons

Das erhaltene Hydroxyester-Rohprodukt (270 g) wurde unter kräftigem Rühren bei 60 °C zu 1080 ml 80%iger Schwefelsäure gegeben. Das Gemisch wurde 1 Stunde bei 60 °C intensiv gerührt und nach Abkühlen auf Eis gegossen und solange nachgerührt, bis das Eis vollständig geschmolzen war. Danach wurde mit Äther extrahiert, die ätherische Phase mit 2n-Sodalösung und anschliessend mit Wasser neutral gewaschen. Nach Trocknen über Natriumsulfat und Abdestillieren des Lösungsmittels hinterblieb das Lacton als kristalline Masse.

c) Herstellung des Diols

Das erhaltene rohe Lacton (204 g) wurde in 3600 ml Isopropanol gelöst. Unter Rühren wurden 45,6 g (1,2 Mol) Natriumborhydrid zugesetzt und das Gemisch wurde 3 Stunden unter Rühren am Rückfluss erhitzt. Nach beendeter Reaktion wurde das Gemisch in die zweifache Menge Wasser eingegossen, das Gemisch mehrfach mit Äther extrahiert, die Extrakte getrocknet, das Lösungsmittel entfernt und das Rohprodukt im Ölpumpenvakuum destilliert.

Das erhaltene Diol stellt eine farblose viskose Flüssigkeit dar mit einem $Kp_{0,6}$ von 172–175 °C.

d) Herstellung des 13,15-Dioxabicyclo[10.5.0]-heptadecan

Das erhaltene Diol (208 g) wurde in 60 ml Äthylenchlorid gelöst, mit 44 g Paraformaldehyd und 1 Tropfen conc. Schwefelsäure versetzt und 3 Stunden lang am Wasserabscheider erhitzt. Nach Beendigung der Reaktion wurden 1,5 g Soda zugegeben, die Hauptmenge des Lösungsmittels bei Normaldruck abgedampft und der Rückstand im Ölpumpenvakuum destilliert. Das erhaltene 13,15-Dioxabicyclo[10.5.0]heptadecan ist eine farblose Flüssigkeit mit folgenden Kennzahlen:

$Kp_{0,6}$ 132 °C

IR (Film): 1000–1170 max. 1060, 1155/cm ($CH-O-CH_2-O-CH_2$)

$^1H$-NMR: $\delta$ 3,68 (m,2H) ($O-C\underline{H}_2-O$); 4,72 (m,3H) ($O-C\underline{H}_2-CH_2$, $C\underline{H}-O-CH_2-O$).

Der Geruch lässt sich als eine warme Ambra-Note, ähnlich gesosstem Tabak, charakterisieren.

Herstellung von 14-Methyl-13,15-Dioxabicyclo-[10.5.0]heptadecan

240 g des wie vorstehend unter c) hergestellten Diols (Formel IV) (1 Mol), 90 g Acetaldehyddimethylacetal (1 Mol) und 0,2 g p-Toluolsulfonsäure wurden 5 Stunden bei 70 °C am Rückfluss erhitzt. Nach beendeter Reaktion wurde das Gemisch in Äther aufgenommen, mit 2n-Sodalösung, danach mit Wasser neutral gewaschen und über wasserfreiem Natriumsulfat getrocknet. Nach Abdestillieren von Äther und Methanol wurde der Rückstand im Ölpumpenvakuum destilliert. Das erhaltene 14-Methyl-13,15-Dioxabicyclo-[10.5.0]heptadecan ist eine farblose Flüssigkeit mit folgenden Kennzahlen:

$Kp_{0,15}$ 119–120 °C

IR-(Film): 1000–1170; max. 1097, 1165/cm ($CH-OCHCH_3-O-CH_2$)

$^1H$-NMR: $\delta$ 3,73 (m,3H) ($O-C\underline{H}_2-CH_2$, $C\underline{H}-O-CHCH_3$) 4,88 (m,1H) ($O-C\underline{H}CH_3$)

Der Geruch lässt sich als warme Ambra-, Holz-, Fruchtnote charakterisieren und ist gegenüber der unsubstituierten Verbindung von deutlich geringerer Intensität.

Beispiel

Riechstoffkomposition Virginia-Tabak-Base

| | Gewichtsteile |
|---|---|
| 13,15-Dioxabicyclo[10.5.0]heptadecan | 150 |
| Boisambrene forte (Henkel KGaA) | 250 |
| Isogeranylnitril | 200 |
| Methyljonon | 100 |
| Vetiverylacetat | 100 |
| 2-Acetyl-4-isopropyl-5,5-dimethyl-1,3-dioxan | 100 |
| Pentadecanolid 10%ig in DEP | 50 |
| Cumarin | 25 |
| Galaxolid (IFF) | 25 |

An die Stelle des 13,15-Dioxabicyclo[10.5.0]heptadecan können auch die in 14-Stellung methylsubstituierten Verbindungen treten.

**Patentansprüche**

1. 13,15-Dioxabicyclo[10.5.0]heptadecane der allgemeinen Formel

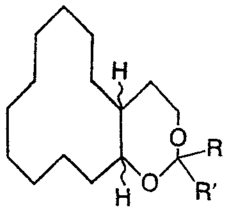

in der R und R' jeweils einzeln oder gemeinsam Wasserstoff oder einen Methylrest darstellen.

2. 13,15-Dioxabicyclo[10.5.0]heptadecan gemäss Anspruch 1.

3. Verfahren zur Herstellung von 13,15-Dioxabicyclo{10.5.0}heptadecan nach Anspruch 2, ausgehend von Cyclododecanon, gekennzeichnet durch eine Reaktionsfolge aus
a) Umsetzung mit Bromessigester zum entsprechenden Hydroxyester,
b) dessen Umlagerung zum Lacton,
c) Überführen des Lactons mittels Natriumborhydrid zum Diol und
d) Acetalisierung des Diols mit Paraformaldehyd zum 13,15-Dioxabicyclo{10.5.0}heptadecan in Form zweier Stereoisomerer.

4. Verwendung von 13,15-Dioxabicyclo{10.5.0}-heptadecanen nach Anspruch 1 und 2 als Riechstoffe.

5. Riechstoffkompositionen, gekennzeichnet durch einen Gehalt an 13,15-Dioxabicyclo{10.5.0}-heptadecanen nach Anspruch 1 und 2 in einer Menge von 1–50 Gewichtsprozent, bezogen auf die gesamte Komposition.

**Claims**

1. 13,15-dioxabicyclo[10.5.0]-heptadecanes corresponding to the following general formula

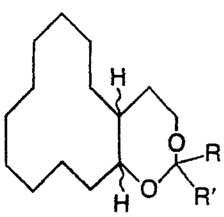

in which R and R' individually or together represent hydrogen or a methyl radical.

2. 13,15-dioxabicyclo-[10.5.0]-heptadecane as claimed in Claim 1.

3. A process for producing the 13,15-dioxabicyclo-[10.5.0]-heptadecane claimed in Claim 2 from cyclododecanone, characterised by a reaction sequence of
a) reaction with bromoacetic acid ester to form the corresponding hydroxy ester,
b) rearranging the hydroxy ester to form the lactone,
c) converting the lactone into the diol using sodium borohydride and
d) acetalising the diol with paraformaldehyde to give the 13,15-dioxabicyclo-[10.5.0]-heptadecane in the form of two stereoisomers.

4. The use of the 13,15-dioxabicyclo-[10.5.0]-heptadecanes claimed in Claims 1 and 2 as fragrances.

5. Fragrance compositions characterised by a content of from 1 to 50% by weight, based on the composition as a whole, of the 13,15-dioxabicyclo-[10.5.0]-heptadecanes claimed in Claims 1 and 2.

**Revendications**

1. 13,15-dioxabicyclo[10.5.0]-heptadécanes de formule générale

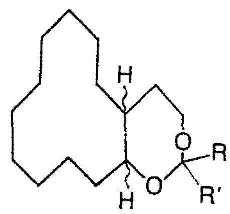

dans laquelle R et R' représentent chacun, individuellement ou en commun, un atome d'hydrogène ou un groupe méthyle.

2. Composé selon la revendication 1: le 13,15-dioxabicyclo[10.5.0]heptadécane.

3. Procédé de préparation du 13,15-dioxabicyclo[10.5.0]heptadécane selon la revendication 2, au départ de la cyclododécanone, caractérisé par la séquence de réactions ci-après:
a) réaction avec le bromacétate d'éthyle, conduisant à l'hydroxyester correspondant,
b) transposition de ce dernier en lactone,
c) conversion de la lactone en diol à l'aide du borohydrure de sodium et
d) acétalisation du diol par le paraformaldéhyde, conduisant au 13,15-dioxabicyclo[10.5.0]heptadécane sous forme de deux stéréo-isomères.

4. Utilisation des 13,15-dioxabicyclo[10.5.0]-heptadécanes selon les revendications 1 et 2 en tant que matières aromatiques.

5. Compositions de parfums, caractérisées en ce qu'elles contiennent des 13,15-dioxabicyclo-[10.5.0]heptadécanes selon les revendications 1 et 2 en quantités de 1 à 50% du poids de la composition totale.